# EUROPEAN PATENT SPECIFICATION

(11) **EP 3 046 779 B1**
(45) Date of publication and mention of the grant of the patent: **08.11.2017**
(21) Application number: 14825192.9
(22) Date of filing: 17.09.2014
(51) Int. Cl.: B44D 7/00, C12R 1/01

(54) **BIOTECHNOLOGY PROCESS FOR THE REMOVAL OF COHERENT DEPOSITS OF ORGANIC AND INORGANIC ORIGIN FROM MATERIALS AND WORKS OF ARTISTIC HISTORICAL INTEREST**
BIOTECHNOLOGISCHES VERFAHREN ZUR ENTFERNUNG VON KOHÄRENTEN ABLAGERUNGEN ORGANISCHEN UND ANORGANISCHEN URSPRUNGS VON MATERIALIEN UND WERKEN VON KÜNSTLERISCHEM UND HISTORISCHEM INTERESSE
PROCÉDÉ BIOTECHNOLOGIQUE POUR L'ÉLIMINATION DE DÉPÔTS COHÉRENTS D'ORIGINE ORGANIQUE ET INORGANIQUE DE MATÉRIAUX ET D'OUVRAGES D'INTÉRÊT HISTORIQUE ARTISTIQUE

(30) Priority: 18.09.2013 IT RM20130519
(43) Date of publication of application: 27.07.2016
(73) Proprietor: ENEA-Agenzia Nazionale per le Nuove Tecnologie, l'Energia e lo Sviluppo Economico Sostenibile, 00196 Roma (RM) (IT)
(72) Inventor: SPROCATI, Anna Rosa, 00061 Anguillara Sabazia (RM) (IT); ALISI, Chiara, 00151 Roma (IT); TASSO, Flavia, 00057 Roma (IT)
(74) Representative: Sarpi, Maurizio
(86) International application number: PCT/IT2014/000246
(87) International publication number: WO 2015/040647

(56) References cited:
- WO-A2-2007/119258
- DE-A1- 10 302 120
- DE-C- 418 696
- FR-A- 459 922
- CAPPITELLI FRANCESCA ET AL: "Improved methodology for bioremoval of black crusts on historical stone artworks by use of sulfate-reducing bacteria", APPLIED AND ENVIRONMENTAL MICROBIOLOGY, AMERICAN SOCIETY FOR MICROBIOLOGY, US, vol. 72, no. 5, 1 May 2006 (2006-05-01), pages 3733-3737, XP002441916, ISSN: 0099-2240, DOI: 10.1128/AEM.72.5.3733-3737.2006
- DAWSON T L: "EXAMINATION, CONSERVATION AND RESTORATION OF PAINTED ART", 1 January 2007 (2007-01-01), COLORATION TECHNOLOGY, SOCIETY OF DYERS & COLOURISTS, BRADFORD, GB, PAGE(S) 281 - 292, XP001511754, ISSN: 1472-3581 the whole document

## Description

The present invention relates to a process for removing different coherent deposits, organic and inorganic, also superimposed, from works of art and/or wall paintings. The process uses, alone or in combination, specific non-pathogenic and non-spore-forming bacterial strains, with aerobic metabolism, immobilized in a synthetic gelling clay, known under the commercial name of laponite. At the end of the treatment the specific biologic composition can be easily removed without affecting the underlying pictorial patina or the constitutive material. The treatment has proved to be safe for the operator, the environment and the work of art.

### Background of the invention

The aim of a restoration is to re-establish the full functionality of a work of art and prolong its conservation. It is a form of activity developed over time according to different requirements, depending on the historical moment, and it is still an evolving matter, open to careful scientific and technical analysis and lively cultural debates.

The field of restoration of wall paintings and stone material is of particular importance.

The term wall paintings comprises all works of art that, although realized by different painting techniques, are performed on a wall support, such as frescoes, half fresco, tempera, graffiti. The restoration of such art works is always performed on site, unless, for particular technical reasons, removing part of the painting from the original support is necessary.

In order to plan a suitable restoration, before starting any intervention, having the full knowledge of the art work on which the action should be taken, analyzing the constituent materials, the execution techniques, the causes of degradation and developing a diagnostic analysis of the state of conservation is pivotal. Stratigraphic assays and cleaning trials of the painting can be carried out to analyze conditions of the painted surface and check out if there are any overlaps. Further in depth examinations can be carried out through chemical-physical analyzes by nondestructive sampling or withdrawal of small samples of the painting to be analyzed in the laboratory or by specific field instruments. If the painting results to be in very precarious conditions, especially at the structural level, adopting safety measures before proceeding with the cleaning of the painted surface is appropriate.

Cleaning of a painting enables the preliminary removal of superficial foreign matter (cobwebs, air dust, surface dirt deposited on the surface of the work over time, stains, drips, splashes) preventing or strongly restricting the correct reading of the work, and, subsequently, the removal of different coherent deposits from the frescoed surfaces, including casein, carbonate, gypsum and phosphate deposits, without spoiling the painted surface. Since coherent substrates strongly adhere to the surface of the work, in order to ensure the best results, cleaning must be selective and performed through the removal of successive layers, distinguishing one level from the other, without removing the original materials to prevent irreversible damage to the work.

Techniques based on chemical methods, often in combination with a mechanical action, are normally used to treat the surfaces of artworks and/or wall paintings. Although in some cases such association has proved to be effective in removing alterations, some disadvantages have been noted. Firstly, the cleaning by chemical methods is usually carried out using chemical solvents and solubilizing agents that sometimes can give unsatisfactory results, due to the low selectivity or high aggression toward the substrates, potentially causing changes in the substrate properties and toxic, both for the environment and the operator. Some traditional cleaning methods for artworks still use toxic organic solvents, such as toluene, xylene, dimethylformamide, dichloromethane. On the other hand, also the mechanical action performed by scalpel, requires a particularly delicate and precise execution to avoid irreparable physical damage to the surface underlying the deposition, and very long execution times, that obliges operators to prolonged permanence in uncomfortable environments, such as caves and tombs.

Recently in the biotechnology field innovative procedures applicable to the characterization of biological deterioration of substrates (biodeterioration) and restoration (biorestoration), have been developed and applied to projects for the conservation/restoration of works of art.

For the biological removal of deposits two approaches are used: the application of purified biological molecules, such as enzymes, and the use of viable bacterial strains, chosen for their selective action towards specific substrates.

The use of purified enzymes is highly selective and quick; it allows to accelerate chemical reactions up to 10⁸ - 10¹⁰. The outcome of enzymatic cleaning depends on many factors, such as the choice of the suitable enzyme, the operating conditions and the delivery system. Optimal pH and temperature of the enzyme activity must be compatible with the material to be treated and requires that proper conditions are maintained throughout the treatment period. Moreover, given the high action specificity of enzymes, in order to achieve satisfactory results, the combination of different enzyme preparations is often necessary. Difficulties in maintaining constant the optimal operating conditions during the application and the high costs make the procedure of enzymatic cleaning critical and not accessible to all restorers.

To overcome these drawbacks approaches based on the use of viable bacterial cells have been developed. The viable cells, as enzymes producers, show huge potentials in the induction of chemical transformations, because they can accelerate the reactions, use the substrate directly as a carbon source, and release active enzymes in the surrounding environment. The application of viable bacterial cells has proved to be more effective in the attack and in the degradation of different types of substrates than direct enzymes. Experience has confirmed bacteria are versatile, have the ability to produce a wide range of enzymes, including inducible enzymes, which are produced by cells only under specific conditions or in the presence of certain substrates.

The main precaution to be considered when applying viable bacterial cells is to use safe microorganisms, not pathogenic or aggressive towards the constituent material and non-spore-forming. This ensures the safety for the operators, the integrity of the works of art, and the correct and complete removal of the bacterial cells can be obtained after the treatment, without leaving quiescent biological elements, which could revitalize when the conditions become favorable again.

The selection of microorganisms suitable to specific application in bio-cleaning treatment and bioremediation of artifacts requires a careful metabolic characterization of the bacterial strains.

The sources of the useful microbial strains are ubiquitous, but often the most critical environmental situations offer the possibility to isolate bacteria able to effectively perform specific functions due to the selective pressure to which they were subjected, i.e. bacteria isolated from contaminated sites, from extreme environments and bio-deteriorated materials of historical and artistic interest are good bio-cleaners. By producing and secreting molecules such as enzymes, organic acids and surfactants, the microorganisms are able to change the chemical structure of organic substrates. Knowing the metabolism of these microorganisms allows to understand how to exploit them, and the knowledge of the mechanism responsible for the degradation of stone materials allows to understand how to favorably exploit deteriogens microorganisms for bio-cleaning applications.

The bio-cleaning protocols exploit both "destructive" action, due to catabolic activity, which can be used for the biological removal of surface coatings on works of art, and "constructive" action of certain microorganisms able to carry out the bio-mineralization process, i.e. to induce the formation of calcite precipitates on carbonate based materials useful for bio-consolidation applications.

As the materials to be removed are in direct contact with the surface of the artwork to be treated, the cleaning has to be carried out in a selective way, through the removal of successive deposit layers, differentiating the action among areas, relieving or removing deeply, without causing irreversible damage. Bio-cleaning seems to meet these accurate criteria, as research in molecular biology and microbiology fields has allowed the selection of several bacterial strains with potential applications in the field of coherent deposits removal from art objects and wall paintings and for bio-deteriogens control as well.

The application of microorganisms or macromolecules thereof, on surfaces, by means of aqueous media is a viable alternative to traditional methods.

The first examples of viable bacteria application for bio-cleaning procedures were performed on stone for the removal of black crusts and salts. In 1970, Moncrieff and Hempel (Moncrieff A., Hempel K. Work on the degeneration of sculptural stone. Conservation of stone and wooden objects, New York Conference, 2nd Ed, ICC, Ed, London, vol. 1, 103-114) have described the use of a biological wet-pack and the role of microorganisms inside of the wet-pack. In order to remove surface alterations of frescoes and works of art due to nitrification, sulphation and black crusts, the use of denitrifying and desulphurising microorganisms applied to the surfaces to be treated has been proposed.

The first successful applications of anaerobic sulphate-reducing bacteria were reported in Atlas RM 1995. Bioremediation. Chem. News 73, 32-42, and Gauri and Chowdhury (KL Gauri, Chowdhury AN 1988 Experimental studies on conversion of gypsum to calcite by microbes: In Proceedings of the 6th International Congress on deterioration and Conservation of Stone. Ciabach J. ed., pages 545-550, Nicholas Copernicus University) wherein the microorganism *Desulfovibrio desulfuricans* were used for the removal of sulphates of black crusts, while *Desulfovibrio vulgaris* was applied on gypsum crusts (Heselmeyer K. et al. 1991, Application of Desulfovibrio vulgaris for the bioconservation of rock gypsum crusts into calcite. BIOforum 1, 89) to obtain, in addition to the removal of blacks deposits, the conversion of gypsum into calcite. According to these techniques marble statues are treated by immersion in a bath of desulphurising bacteria in order to eliminate the gypsum deposits (Gauri et al., 1989 The sulphation of marble and the treatment of gypsum crust. Stud. Cons. 34:201-206). *Pseudomonas* stutzeri and P. aeruginosa are the denitrifying bacteria employed for the removal from objects of art, respectively sulphates and nitrates (Ranalli et al. 1997. The use of microorganisms for the removal of sulphates on artistic stoneworks. International biodeterioration and biodegradation, 40:255-261). However, due to the generally large size of the object to be treated this type of application presents disadvantages associated with the volume of the treatment bath. Consequently, this treatment is not suitable for application to buildings and frescoes, and also requires a consolidation phase of the work to prevent serious damage caused by the bath immersion.

Alternatively, the use of a supporting matrix has been proposed, such as sepiolite, to trap microorganisms and apply said matrix to the object to be treated. This method requires a long and laborious preparation, at least a week to allow bacterial growth and colonization of the sepiolite particles. Furthermore, the system does not maintain favorable conditions over time, namely the water availability, so the trapped bacteria can efficiently perform their action and a high concentration of microorganisms is required to survive to the adverse conditions.

The use of organic media, such as polyacrylic acid, has been proposed to overcome the problems associated with microbial colonization of inorganic supports. During the gel preparation microorganisms are trapped in the organic gel, the procedure takes about 10 minutes, providing a considerable advantage compared to inorganic supports requiring at least 7 days to grow and adhere on the particles of the inorganic matrix. However, organic supports, such as polyacrylic acid, have a limited ability to adhere to any type of stone or metal.

In the restoration of polychrome works, several protocols allowing to modulate the microbial metabolism and the action of hydrolytic enzymes have been developed. Recently, Sorlini and collaborators have restored a fresco of the Monumental Cemetery of Pisa, "Conversione di S. Efisio e battaglia". The fresco resulted to be irreparably damaged by a previous treatment with formaldehyde and glue. In this last restoration viable bacterial cells, combined with specific enzymes degrading the organic matrix of the animal glue, have been used. Namely, the used *Pseudomonas* stutzeri A29 strain is able to quickly degrade the glue, with minimal risk of pigments alteration, and efficiency between 80-90%. The operation was performed on different areas of the fresco, keeping the bacteria on the surface for 10-12 hours at 28-30 °C. Bacteria degraded most of the organic substance, while the residual glue was removed by "Protease Type XIX". At the end of the treatment the bacteria have to be removed from the surface to avoid possible pigments deterioration.

Above and below the frescoed surface, water diffusion or absorption of the aqueous media delivering the bio-cleaning media can damage the work, therefore a control system to limit the water intake in contact with the top layer of the work has to be developed, and the synergistic action of the support matrix for trapping and applying microorganisms to the object to be treated is an important factor.

Such control action may be achieved by adding substances, i.e. mineral or organic thickeners or gelling agents increasing the solution viscosity. A 1-1.5 % (w/v) polyacrylic acid composition, whose formulation is known under the commercial name Carbopol, can produce very high viscosity and is easily removable. However, it is acidic and becomes a gel only after neutralization with a base, such as triethanolamine, or sodium or ammonium hydroxides, hence it permeates the work of art surface.

The use of gelling agents well known in the food industry and bio-medical field have been recently tested to solve this problem. Agar and agarose have been used as support in cleaning and bio-cleaning applications. The peculiar gelling properties of such molecules provide them with the ability to retain high amounts of water in their mesh. Contacting porous materials, such as painted surfaces, statues or paper, these rigid gels can gradually release water and absorb dissolved deposits to be removed. The gel solidity allows its easy removal after use, leaving no residue on the surface. Another significant feature of agar and agarose gel is the reversibility, since they melt by heating and harden by cooling. This feature also enable the application of gel on rigid non-planar surfaces, such as statues, models of plaster and stucco, by applying the molten gel and letting it cool, so that it can take the form of the object surface. For specific cleaning tasks, an aqueous solution of chelating agents, surfactants or enzymes can be added to the gel.

The patent publication WO 2007/119258 A2, claiming priority from application MI2006A000776, entitled:
"Process for the bio-cleansing of the surfaces of objects of various chemical natures and buildings" discloses the use of some bacterial strains of the Desulsovibrio genus for removing sulphates deposits from stone surfaces and strains of denitrifying bacteria, selected in the *Pseudomonas stutzeri* species, for cleaning objects of various chemical nature and buildings, involving an inert material as a means of support for the growth of microorganisms, able to maintain the necessary and sufficient conditions to let them fulfill the bio-cleaner role. According to the invention the supporting inert material is a matrix comprising a polymeric organic compound which is dispersed in a silica or a silicate-based inorganic compound, such as sepiolite, upon which the bacterial biofilm grows. The formulation is applied through a spatula in a 0.5-3 cm thick layer. In order to improve the retention of the water contained in the organic polymer, and reduce the oxygen diffusion, in case of anaerobic microorganisms, the biological formulation can be covered with a plastic elastic film to improve the anaerobic microorganisms action. After application, the organic formulation is left in position for a time ranging between 6 hours and 7 days, more preferably between 8 and 72 hours, depending on the type of microorganism or the chemical conditions of the object to be treated. Several applications in succession can be performed.

The successful use of rigid gel as support for cleanup operations has recently led to the development of new types of gel, such as reactive chemical magnetic hydrogels, tested on murals (Bonini et al. 2008. Acrylamide-Based Magnetic Nanosponges: A new smart nano composite material: Langmuir 24, 12644-12650).

However, in case of unfavorable environmental conditions for the temperature and in case of rain, the application of bio-cleaning techniques, may require the adoption of simple expedients, such as the application of a black plastic film, possibly in association with a heating lamp to balance any temperature drop. In the driest climates several repeated application cycles allow to administer fresh layers of formulation, improve moistening and vary the amount of organic component. In outdoor objects, devices and solutions preventing runoff caused by rain and other adverse events (wind, snow, etc.), or fixed cover (roofs, porches portable or fixed, etc.) have to be developed and implemented.

Although these devices and solutions fall within the scope of the knowledge and skills of the expert of the sector, they complicate the application of bio-cleaning methods.

Therefore, the need to develop alternative bio-cleaning processes through microbial strains providing different application modes than those described above, allowing essentially a simpler administration, less susceptible to environmental variables, and also ensuring more reproducible results in a wider range of applications, is strongly felt. Such higher degree of reproducibility of the final result is also due to the particular selected bacterial strains that in the present invention act within a particular support matrix, removing in a synergistic way coherent deposits of different nature.

### Summary of the Invention

In a first aspect the present invention provides a process for removing coherent deposits of various nature, organic and inorganic, from objects of art and/or mural paintings using new, non-pathogenic and non spore forming bacterial strains, immobilized within an inert support in the form of gel. The new bacterial strains were selected for their ability to degrade specific organic substrates, such as casein, and solubilize inorganic deposits (chalk, carbonates and phosphates) under aerobic conditions.

A second aspect of the invention is represented by the three new bacterial strains, isolated from soils and underground environments, used in the process for removing coherent deposits of various nature.

A third aspect of the invention is the original formulation constituted by the association of the bacterial strains with the specific gel matrix.

A further aspect of the invention consists in the kit for the bio-cleaning of objects of art and/or mural paintings from coherent deposits comprising the association of one or more bacterial strains with the specific gel matrix.

### Detailed description of the invention

The present invention provides a process for removing coherent deposits of various nature, organic and inorganic, from objects of art and/or mural paintings. The method uses three new bacterial strains immobilized in a specific gelling matrix.

Such gelling matrix is an industrial synthetic clay belonging to the smectite family, the product is commercially known as laponite. It is a hydrated magnesium silicate layered, hydro-thermally synthesized by mixing silicate, lithium and magnesium salts, in the presence of mineralizing agents. The chemical formula that describes such synthetic clay, providing support to the bacteria, is: Na_{0.7}[(Si₈Mg_{5.5}Li_{0.3})O₂₀(OH)₄] (Daniel L.M. et al. J. Colloid Interface Sci. 2007, 316: 72-79).

The interaction of organic molecules with the acidic and hydrophilic clay surface can cause enzyme denaturation, therefore its use in the biotechnology field is problematic (Tietjen T, Wetzel RG. Extracellular enzyme-clay mineral complexes: Enzyme adsorption, alteration of enzyme activity and protection from photodegradation. Aquatic Ecology. 2003; 34(4):331-339). Such limitations can be overcome through the passivation process of the acid surface sites and creating a more organophilic clay matrix, with different capabilities.

In the present invention no chemical-physical process has to be applied to the clay composition, in order to make it compatible with the biological systems to be included, because the use of live bacterial cells overcome the incompatibilities known for bio-molecules.

The approach described in the present invention represents a further possible alternative to overcome the limitations of the applications of this synthetic clay in the field of biotechnology.

The bacterial strains used in this invention were isolated by the Applicant for the first time from soil and underground environments, and were selected in the laboratory for their high capacity to degrade specific organic substrates, such as casein, and to solubilize inorganic deposits, such as chalk, carbonates and phosphates, in aerobic conditions. Then, for purposes of patent procedure, in compliance with the provisions according to the Budapest Treaty, the Applicant has deposited the three bacterial strains with the general collection Deutsche Sammlung von Mikroorganism und Zellkulturen GmbH, Braunschweig, Germany.

A first bacterial strain used in the process according to the invention, *Stenotrophomonas maltophilia UI3E,* was filed by the Applicant on May 17, 2013 at the Deutsche Sammlung von Mikroorganismen Und Zellkulturen GmbH, Braunschweig, Germany, which has been associated with identification number DSM 27225.

The genus Stenotrophomonas comprises at least eight species. These bacteria are ubiquitous in aqueous environments, in soil and in particular in association with plants. The strains of the predominant species, *Stenotrophomonas* maltophilia, have an extraordinary range of activities including the beneficial effects on growth and health of plants, degradation of xenobiotic and natural pollutants, important for environmental remediation strategies, and the production of biomolecules of economic value. On the other hand, there are negative effects such as multi-drug resistance in strains pathogenic for humans. (Robert P. Ryan, Sebastien Monchy, Massimiliano Cardinale, Safiyh Taghavi, Lisa Crossman, Matthew B. Avison, Gabriele Berg, Daniel van der Lelie & J. Maxwell Dow. The versatility and adaptation of bacteria from the genus Stenotrophomonas Nature Reviews Microbiology. 7 2009, 514-525).

*Stenotrophomonas maltophilia* is an aerobic, non-fermentative, Gram-negative bacterium. The cells are slightly smaller (0.7-1.8 x 0.4-0.7 µm) than other members of the genus. They are mobile thanks to polar flagella and grow well on MacConkey medium, producing pigmented colonies. S. *maltophilia* are catalase-positive, oxidase-negative, this feature distinguishes it from other members of the genus, and has a positive reaction to the extracellular DNase.

S. *maltophilia* is ubiquitous in aqueous environments, in soil and plants, but has also been used in biotechnological applications (G. Berg et al. 1999. "Genotypic and phenotypic relationships between clinical and environmental isolates of Stenotrophomonas maltophilia." J Clin Microbiol 37: 3594-600), and in agriculture for the biological control of plant pathogenic fungi (G. Berg et al., Stenotrophomonas maltophilia in the rhizosphere of oilseed rape-occurrence, characterization and interaction with phytopathogenic fungi. Microbiol Res. 1996; 151 : 19-27), in bioremediation (X. Wang et al. A three-component enzyme system catalyzes the demethylation of the herbizide dicamba in Pseudomonas maltophilia DI-6. Appl Environ Microbiol. 1997; 63: 1623-1626). Being an opportunistic pathogen in immuno-depressed patients, S. *maltophilia* can lead to nosocomial infections.

The new strain of the present invention, *Stenotrophomonas maltophilia UI3E,* was isolated from the rhizosphere of a mining site located in Ingurtosu, Sardinia, Italy. Morphological analysis shows rod cell from 2 to 2.5 µm in length, and 0.35 µm in width. The strain identification and classification was performed by analysis of the partial sequence (1242 base pairs) of the 16S rRNA gene compared with the sequences deposited in the NCBI database (GenBank) with a similarity index of 99%, and through metabolic characterization by Biolog™ system. As described below, this strain has demonstrated in vitro the ability to degrade specific organic substrates such as casein.

A second bacterial strain used in the process according to the invention belongs to the genus Cellulomonas (now Cellulosimicrobium). The genus Cellulosimicrobium comprises gram-positive, filamentous and branched, bacilli. One of the specific features of the bacteria belonging to this genus is their ability to degrade cellulose, using specific enzymes such as endoglucanases and esoglucanases

The new strain of the invention, *Cellulomonas sp. TBF11E* was filed by the Applicant on May 17, 2013 at the Deutsche Sammlung von Mikroorganismen Und Zellkulturen GmbH, Braunschweig, Germany, which has been associated with identification number DSM 27224.

The new strain *Cellulomonas sp. TBF11E,* was isolated and selected from over one hundred strains obtained from the walls of the inner chamber of an Etruscan tomb, located in Tarquinia, Lazio, Italy.

It is a Gram positive bacterium type rod shape ranging from 1 to 3 µm in length, and 0.4 µm in width. The strain identification and classification was performed by analysis of the partial sequence (1282 base pairs) of the 16S rRNA gene compared with the sequences deposited in the NCBI database (GenBank) and through metabolic characterization by Biolog™ system.

As demonstrated below, this strain shows to have a high capacity to solubilize inorganic deposits, such as gypsum and carbonates, in normal environmental conditions.

A third object of the present invention is the bacterial strain *Pseudomonas koreensis UT30E,* also filed by the Applicant on May 17, 2013 at the Deutsche Sammlung von Mikroorganismen und Zellkulturen GmbH, Braunschweig, Germany, identified by the number DSM 27226.

Pseudomonas is a bacterial genus having the highest diversity, and its taxonomy has undergone several changes since its first descriptions (Palleroni, Prokaryote taxonomy of the 20th century and the impact of studies on the genus Pseudomonas: a personal view 2003 Microbiology 149: 1-7). Furthermore, the number of species affiliated to the genus Pseudomonas is increasing every year, they were 102 in 2006, 109 in 2007, 114 in 2008, 118 in 2009, counting 205 in 2013 (http://www.bacterio.cict.fr/ and http://www.dsmz.de/). Pseudomonas bacteria are Gram-negative, non-spore-forming, obligate aerobes, oxidase positive, catalase positive and have polar flagella that allow them to move. They are found in soil and water, but also on the plants. Some strains are producers of natural antibiotic substances and are used in agriculture for the control of phytopathogens (Ps. aurantica), other strains, able to degrade hydrocarbons, have been used to clean polluted environments (Ps. putida).

The original strain of the present invention was isolated from the rhizosphere of the mining site of Trezbionka, Poland. Cell morphological analysis has revealed a typical rod shape 1 - 2 µm in length, and 0.3 3 µm in width. The strain is able to solubilize phosphates based deposits in aerobic conditions.

The identification and classification of the strain was performed and confirmed by sequence analysis of 16S rDNA gene (1399 bp) compared with the sequences deposited in the NCBI database (GenBank) with a similarity index of 99%, and through metabolic characterization by Biolog™ system.

The propagation of the three strains can be carried out by culture under standard conditions and in the conventional media used for bacterial cells growth. Several culture media are well-known to be suitable for the use according to the invention. Namely, and without being limited to this, the three strains are grown in liquid cultures in aerobic media containing sources of carbon, nitrogen and inorganic salts. The preferred carbon source are carbohydrates, but other sources include glycerol, amino acids, xylose, etc... Many inorganic materials and protein can be used as a nitrogen source in the growth process. Preferred source of nitrogen are amino acids and urea, but they also include inorganic salts of nitrate and ammonium; other sources of metabolites are vitamins, purines, pyrimidines, yeast extract, peptone, soybean meal, hydrolyzed casein, and the like. Inorganic minerals that can be incorporated in the growth medium include calcium, zinc, iron, manganese, magnesium, copper, cobalt, potassium, sodium, molybdate, phosphate, sulfate, chloride, borate, in ionic form as well.

Similarly, pH values and temperature are variable, and the optimum conditions may vary depending on the particular type of bacteria. However, the cell growth according to the invention can be performed at temperatures between 6 and 37 °C, the preferred temperature range is 25 to 30 °C.

The pH value in the culture medium can vary between 4 and 9, but the optimum range is 6-8. The bacteria must be grown under aerobic conditions, preferably under agitation. The harvest time is dependent on strain and growing conditions, in particular on culture medium, temperature and aeration. For illustrative purposes and without being limiting, the cultures of the bacterial strains are typically harvested 72 hours after inoculation, when grown at 25 °C, but may be collected as early as 20 to 24 hours, especially when grown under conditions which determine a more rapid growth, such as a higher temperature (26-30 °C) or in some medium containing ingredients that are metabolized more rapidly.

In a particularly preferred embodiment of the invention, the bacterial strains grow in TSB culture medium (Tryptic Soy Broth) or on solid medium plate in TSA (Tryptic Soy Agar) at the optimal temperature of 28 °C for 24-48 hours.

The resulting cultures are recovered for subsequent use in the process according to the invention to form a composition including a matrix providing a support to microorganisms.

According to the present invention, the support for the bacterial strains that metabolize deposits is provided by the use of a synthetic colloidal clay, known by the commercial name of laponite and usually used as an additive in various products in order to modify the rheology and as a trainer of film to create antistatic barriers. Such clay appears as a fine white powder; if dispersed in tap water it swells and forms in less than an hour a highly thixotropic gel, while, at the same concentration in distilled water, only provides a limited viscosity. The viscosity of the laponite dispersions depends on the content of electrolytes present in the water.

The commercial synthetic clay used in the present invention is obtained by the combination of salts of sodium, lithium and magnesium with sodium silicate at controlled temperatures. The cells that compose it have a characteristic disc shape. A cell is constituted by a layer of magnesium ions sandwiched between two layers of silicon, all balanced by oxygen atoms and hydroxyl groups. During the dispersing process crystals form aggregates held together by electrostatic bonds hence forming an electric double layer. In the presence of salt the crystals take on a structure similar to a "house of cards", responsible for the characteristics of the thixotropic gel.

In fact in the restoration the large retention capacity of laponite toward water is exploited, which allows longer contact time with a limited absorption by the support, a desirable circumstance especially when porous, hygroscopic, soluble or otherwise sensitive materials/surfaces to the action of water, as a fresco, has to be treated. In the restoration, the laponite is used to remove or make less visible rust stains and iron deposits from frescoes, sometimes in combination with citric acid and sodium citrate, but often only with water, to avoid further damage to particularly compromised substrates.

For the first time in the present invention is proposed the combination of specific bacterial strains and the synthetic clay, corresponding to chemical formula Na_{0.7}[(Si₈ Mg_{5.5} Li_{0.3}) O₂₀(OH)₄], known as laponite and the suspension of laponite in a solution other than water.

In fact, the process of removing from objects of art and/or mural paintings different kind of coherent deposits, both organic and inorganic, subject matter of the present invention, according to the procedure as described below involves the use of the bacterial strains in association with a matrix of laponite, following a careful analysis adapted to identify the type of deposits to be removed:
Step A: The bacterial strains are grown in rich culture medium (TSB) with constant stirring (150 rpm) at 28 °C, until reaching the maximum optical density value. (plateau phase).
Step B: The liquid cultures are subjected to centrifugation (6000 rpm for 20 minutes) to separate the cells from the culture medium.
Step C: The cells are then resuspended in a liquid medium with variable composition according to the strain and the type of application it is intended for:
   C1) for the removal of casein deposits, cells of the *Stenotrophomonas maltophilia UI3E* (DSM 27225) strain are resuspended in a 0.5% sodium pyrophosphate solution (w/v).
   C2) for the removal of carbonate and gypsum deposits, cells of the strain *Cellulomonas sp. TBF11E* (DSM 27224) are resuspended in TSB medium diluted in deionized water (1:10 v/v).
   C3) for the removal of phosphate based deposits, cells of the *Pseudomonas koreensis UT30E* strain (DSM 27226) are resuspended in TSB medium diluted in deionized water (1:10 v/v).
Step D: To the so obtained cell suspension the synthetic clay Na_{0.7}[(Si₈Mg_{5.5}Li_{0.3})O₂₀(OH)₄], i.e. the commercial Laponite®RD (Rockwood Additives, CAS No. 53320-86-8), is added at a concentration 5 to 15% (w/v), preferably between 7 and 11% (w/v), even more preferably the laponite is added at a concentration of 9% (w/v).
Step E: The gel containing the bacterial cells is left to stand at a temperature of 4 °C for 16-20 hours, to allow full swelling of clay, irreversible when it returns to room temperature.
Step F: The gel with bacterial cells is applied using a spatula on the surfaces to be treated, namely even onto vertical wall surfaces or ceilings due to its perfect adhesiveness. According to the invention, the gel layer, comprising bacterial strain cells and laponite, is uniformly applied, with a thickness ranging from 0.3 to 1.0 cm. The gel is left to act for at least 24 hours at room temperature.
Step G: At the end of treatment, the gel is removed using a spatula.

In case of organic deposits removal, as described in step C1, the step E serves to bring the cells of *Stenotrophomonas maltophilia UI3E* (DSM 27225) in a state of starvation characterized by a state of lack of source of energy, nutrients and vitamins, which is a physiological condition that predisposes the organism to attack the organic matter to be removed, as the sole source of carbon and energy available, having exhausted during starvation endogenous cellular reserves. Otherwise, any reserves of carbon sources and nutrients resulting from the growth medium, or accumulated in the cells, are disadvantageous for an immediate attack of the substrate to be removed, until the depletion of reserves.

On the contrary, the strain *Cellulomonas sp. TBF11E* (DSM 27224) used for the removal of carbonate and gypsum deposits (C2), and the strain *Pseudomonas koreensis* UT30E (DSM 27226) used for the removal of phosphate deposits (C3) have to be added at this stage with a diluted medium to sustain the bacterial metabolism and allow the solubilization of inorganic deposits.

In a preferred embodiment of the invention, in order to avoid a too fast desiccation of the laponite gel comprising the bacterial suspension, especially in particularly hot and dry environmental conditions, the gel once applied to the site to be treated may be coated with a plastic film. However, the effectiveness of the method according to the invention at temperatures between 6 and 37 °C has been demonstrated. Cells immobilized in 9% laponite (w/v) were viable up to 4 days after the formation of the gel.

At the end of the treatment the gel is easily removed by a spatula, or a similar instrument, without the operation damaging the underlying pictorial patina, having the foresight to collect the gel fragments in a container that can be disposed with a simple sterilization and subsequent disposal as home waste.

Once the gel is removed, the coherent deposit appears reduced and softened so that it can be easily removed with a water dampened sponge and a brush.

The main advantage of the present invention compared to similar compositions of the state of the art useful for the bio-cleansing of wall works of art consists in using aerobic bacterial strains, non-spore-forming, and thus, not leaving dormant forms as spores on substrates, furthermore they are not pathogenic and are employed in not toxic aqueous solutions.

Another advantage of the method of the present invention consists in being able to be applied to the removal of different kind of deposits. This feature is achieved by using three types of bacterial strains with different metabolic competences, which can be used individually or in varying combination between each other depending on the type of action required (either solubilizing or degradative) to perform the best removal of deposits, which are often formed by accumulations and layering of different nature. Alternatively, and in any case always depending on the type of required action, the treatments with the bacterial strains can be repeated in succession. In this way the method is a very versatile tool that can be used to fit the actual condition of the surface to be treated. Therefore, in order to obtain the best results, the chemical analysis of the deposits present on the surface to be treated is very important because it allows the operator to take decisions and select the suitable metabolic attack.

Compared to other known procedures of bio-cleansing of works of art, the present invention makes use of aerobic bacterial strains. This feature provides the advantageous technical effect of not requiring special precautions in the preparation and application of bacteria, because the presence of oxygen, and thus air, does not inhibit the action thereof. Unlike the anaerobic bacteria so far used in the bio-cleansing, the ability to work under normal environmental conditions is an important simplification of the process that makes it accessible to non-specialist operators.

The microorganisms can be stored at -80 °C in a suitable storage medium comprising glycerol, or at room temperature in lyophilized form. Before use they are re-hydrated and reconstituted in an aqueous solution, able to restore the vitality. According to the invention, at the time of use the gel to be applied on the surface is obtained by laponite addition, preferably at a concentration of 9% w/v.

A further object of the invention is a kit for the bio-cleansing of objects of art and/or wall paintings from coherent deposits comprising sufficient material for an application on a given unit of surface area:
- Test tubes containing each bacterial strain in a stabilized suspension, for example, in TSA, or in lyophilized form, as previously described;
- Vials containing aliquots of the culture medium for optimal growth of each of the three strains (i.e. TSB) lyophilized in an amount sufficient for one application;
- Vials containing aliquots of sterilized deionized water in an appropriate amount to dissolve the contents of one vial of culture medium;
- Sachets containing single-use laponite, as previously described, in am amount sufficient for one application.
- Means for applying the suspension of microorganisms in laponite on the surface to be treated;
- Explanatory leaflet with instructions for using the kit.

The application means according to the invention are spatulas, small brushes and sponges.

Further aspects and embodiments of the invention will now be illustrated by the following examples provided for non-limiting purposes.

### Experimental part

### EXAMPLE 1

### Isolation of bacterial strains

Underground sites, such as rock-hewn churches and places of underground worship, represent specific ecological niches where environmental conditions such as light, moisture, temperature, nutrient input, and the porous nature of the substrate become favorable factors and determinants for microbial colonization, often responsible for biodeterioration phenomena that occur on the works of art (Saiz-Jimenez C., "Biodeterioration vs Biodegradation: the role of microorganisms in the removal of pollutants deposited on historic buildings" in "International biodeterioration and biodegradation", 1997, 225-232. Webster A., May E., "Bioremediation of weathered-building stone surfaces", in "TRENDS in Biotechnology", 2006, 255-260).

Following sites inspection, some features of the walls and ceilings of interest because of observed coating or fouling have been visually identified, in this way the areas where sampling was done have been identified, according to the protocol NORMAL 3/80 (1980). Sampling was done by rubbing five sterile swabs over 10x15 cm areas for each sampling area. The samples, in a refrigerated box at 4 °C, were transported to the laboratory and processed the same day.

From the site of an Etruscan tomb located in Tarquinia, Italy a total of 10 areas were sampled. Other samplings were carried out from 10 soil samples of the mining site of Ingurtosu, Italy, and 10 soil samples from the mining site of Trezbionka, Poland. These sites host microorganisms with specific metabolic functions of biotechnological interest, developed as an adaptive response to conditions of chronic stress.

The selection of microbial strains able to solubilize calcium carbonate was performed on TSA agar medium on which a suspension of CaCO₃ 1% at pH 8.4 was distributed. The solubilization test, performed according to the method NORMAL 9/82, 1982 - Microflora autotrofa e eterotrofa: tecniche di isolamento in coltura. CNR-ICR, Comas Graphics, Rome, has allowed to identify microorganisms able to form a halo of carbonate clarification.

A similar method was considered valid to assess the bacteria ability to solubilize plaster.

Petri dishes containing TSA and glazed surface with a suspension of 5% [CaSO₄•2(H₂O)] were prepared. After seeding in spot, plates were left at room temperature (22-25 °C) up to the formation of a halo of clarification of chalk.

The selection of microbial strains able to solubilize phosphate salts was carried out on Pikovskaya medium. The solubilization test, performed according to the Pikovskaya RI method (Mobilization of phosphorus in soil in connection with the vital activity of some microbial species. Mikrobiologiya, 1948, 17: 362-370) has allowed to identify microorganisms able to form a halo after incubations up to 14 days.

The selection of the bacteria able to remove casein deposits was made by assaying the production of extracellular proteases of the isolated strains; for this purpose reference is made to the method described by Vermelho & al. (1996) with modifications.

Briefly, Petri dishes containing TSA supplemented with 1% w/v gelatin (bovine collagen) were prepared. Plates were incubated at 28 °C in a thermostat and observed every 24 hours for 7 days. After this incubation period, plates were stained with a solution of 0.1% black starch in methanol-acetic acid-water, in the ratio 30:10:60 (v/v/v), and incubated at 28 °C for about 2 hours, then the excess solution was removed and a de-staining solution of methanol-acetic acid-water, in the ratio 30:10:60 (v/v/v) was poured. The proteolytic action is assessed on the basis of the formation of a non-colored halo around the colony.

### EXAMPLE 2

### Growth conditions

Each selected strain's optimal growth conditions have been identified.

In particular, *Stenotrophomonas maltophilia UI3E* (DSM 27225) and *Pseudomonas koreensis* UT30E (DSM 27226) grow on solid (TSA) or liquid (TSB) medium at the optimal temperature of 28 °C for 24 hours.

*Cellulomonas sp. TBF11E* (DSM 27224) grows at the same temperature and nutrients conditions but requires longer incubation times, up to 48 hours.

### EXAMPLE 3

### DNA extraction and PCR amplification

The identification of the strains was performed by extracting genomic DNA from individual colonies, according to standard protocols, and amplifying the 16S rRNA gene by PCR (Gradient Euroclone One thermocycler-Euroclone, Milan, Italy) using the universal primers for Eubacteria:
P0 (5'-GAG AGT TTG ATC CGT GCT CAG-3'), and
P6 (5'-CTA CGG CTA CCT TGT TAC GA-3').

All primers were purchased from PRIMM (Milan, Italy).

Aliquots of 2 µl of cell lysates (obtained from a single colony), 25 µl of 2x Master Mix (Bioline, London, UK), and 0,5 µM of each primer were used in PCR reaction (50 µl). The PCR program consists of:
1 min at 95 °C, followed by 30 s at 95 °C, 30 s at 60 °C, 4 min t 72 °C (5 cycles); then 30 s at 95 °C, 30 s at 55 °C, 4 min at 72 °C (5 cycles); then 30 s at 95 °C, 30 s at 50 °C, 4 min at 72 °C (25 cycles), followed by a final cycle at 72 °C (10 min) and 60 °C (10 min).

The amplification products were separated onto 1.0% agarose gel, stained with GelRed (Biotium, Hayward, CA) and visualized with BioRad Gel Doc 2000 Image Analyzer. All DNA framments were purified and concentrated on CL6B200 Sepharose (Sigma, St. Louis, MO).

### EXAMPLE 4

### Sequencing

The sequencing of the 16S rRNA gene was performed by Genechron (ENEA-Casaccia, Rome, Italy) using the same primers used for amplification. The analysis of the sequence and the comparison with those present in the GeneBank database allowed the taxonomic affiliation of the new strains indicated as DSM 27224, DSM 27225 and DSM 27226, respectively, to the species *Cellulomonas sp., Stenotrophomonas maltophilia* and *Pseudomonas koreensis* with 99% homology level for all strains.

### EXAMPLE 5

### Metabolic characterization

The metabolic characterization of the three strains was performed by inoculating the appropriate. micro plates Biolog® (Microstation System 4.2, Biolog Inc., CA) (GN2 for the Gram-negative strains, GP2 for the Gram-positive) and following up the reaction development every 4-24-48 hours by the Microplate Reader spectrophotometer (dual wavelength date: OD590-OD750).

### Results:

Strain *Pseudomonas koreensis UT30E* (DSM 27226): metabolic profile

**MicroPlate GN2**

| Substrate | Reaction | (OD) |
|---|---|---|
| Water | - | 0 |
| a-Cyclodextrin | - | -11 |
| Dextrin | - | 96 |
| Glycogen | - | 29 |
| Tween 40 | + | 199 |
| Tween 80 | B | 171 |
| N-Acetyl-D-Galactosamine | - | 6 |
| N-Acetyl-D-Glucosamine | - | 30 |
| Adonitol | - | 1 |
| L- Arabinose | + | 555 |
| D-Arabitol | + | 264 |
| D-Cellobiose | - | 3 |
| i-Erythritol | - | -5 |
| D-Fructose | + | 180 |
| L-Fucose | - | 4 |
| D-Galactose | + | 390 |
| Gentiobiose | - | 4 |
| a-D-Glucose | + | 364 |
| m-Inositol | - | -2 |
| a-D-Lactose | - | 3 |
| Lactulose | - | 1 |
| Maltose | - | 1 |
| D-Mannitol | + | 289 |
| D-Mannose | + | 211 |
| D-Melibiose | - | 1 |
| b-Methyl-D-Glucoside | - | 1 |
| D-Psicose | - | 48 |
| D-Raffinose | - | -2 |
| L-Rhamnose | - | 5 |
| D-Sorbitol | - | 5 |
| Sucrose | - | 1 |
| D-Trehalose | - | 0 |
| Turanose | - | 3 |
| Xylitol | - | 4 |
| Pyruvic Acid Methyl Ester | + | 188 |
| Succinic Acid Mono-Methyl Ester | + | 209 |
| Acetic Acid | + | 307 |
| Cis-Aconitic Acid | + | 592 |
| Citric Acid | + | 799 |
| Formic Acid | - | 62 |
| D-Galactonic Acid Lactone | + | 211 |
| D-Galacturonic Acid | - | 6 |
| D-Gluconic Acid | + | 317 |
| D-Glucosaminic Acid | - | 13 |
| D-Glucuronic Acid | - | 8 |
| α-Hydroxybutyric Acid | B | 140 |
| β-Hydroxybutyric Acid | + | 329 |
| γ-Hydroxybutyric Acid | - | 9 |
| p-Hydroxyphenyl-acetic Acid | - | -10 |
| Itaconic Acid | - | -8 |
| α-Ketobutyric Acid | - | 77 |
| α-Ketoglutaric Acid | + | 534 |
| α-Ketovaleric Acid | B | 106 |
| D,L-Lactic Acid | + | 383 |
| Malonic Acid | + | 223 |
| Propionic Acid | + | 221 |
| Quinic Acid | + | 227 |
| D-Saccharic Acid | + | 549 |
| Sebacic Acid | - | 5 |
| Succinic Acid | + | 416 |
| Bromosuccinic Acid | + | 435 |
| Succinamic Acid | B | 114 |
| Glucuronamide | - | 1 |
| L-Alaninamide | B | 132 |
| D-Alanine | + | 232 |
| L-Alanine | + | 210 |
| L-Alanyl-Glycine | + | 247 |
| L-Asparagine | + | 426 |
| L-Aspartic Acid | + | 415 |
| L-Glutamic Acid | + | 319 |
| Glycyl-L-Aspartic Acid | - | -1 |
| Glycyl-L-Glutamic Acid | + | 267 |
| L-Histidine | - | 6 |
| Hydroxy-L-Proline | + | 296 |
| L-Leucine | B | 161 |
| L-Ornithine | - | 52 |
| L-Phenylalanine | - | 14 |
| L-Proline | + | 208 |
| L-Pyroglutamic Acid | + | 207 |
| D-Serine | - | 45 |
| L-Serine | + | 240 |
| L-Threonine | - | 85 |
| D,L-Carnitine | + | 209 |
| γ-Aminobutyric Acid | + | 231 |
| Urocanic Acid | + | 334 |
| Inosine | + | 324 |
| Uridine | - | 60 |
| Thymidine | - | -1 |
| Phenylethylamine | - | 4 |
| Putrescine | B | 98 |
| 2-Aminoethanol | B | 161 |
| 2,3-Butanediol | - | -1 |
| Glycerol | + | 185 |
| D,L-α-Glycerol Phosphate | + | 204 |
| α-D-Glucose-1-Phosphate | - | -3 |
| D-Glucose-6-Phosphate | - | -3 |

Strain *Cellulosimicrobium cellulans TBF11E* (DSM 27224): metabolic profile

**MicroPlate GP2**

| Substrate | Reaction | (OD) |
|---|---|---|
| Water | - | 0 |
| α-Cyclodextrin | + | 1126 |
| β-Cyclodextrin | + | 881 |
| Dextrin | + | 1494 |
| Glycogen | + | 1578 |
| Inulin | - | 107 |
| Mannan | + | 1391 |
| Tween 40 | + | 1023 |
| Tween 80 | - | 360 |
| N-Acetyl-D-Glucosamine | + | 855 |
| N-Acetyl-β-D-Mannosamine | + | 820 |
| Amygdalin | + | 1102 |
| Arabinose | + | 1413 |
| Arabitol | + | 864 |
| Arbutin | + | 1110 |
| Cellobiose | + | 1511 |
| Fructose | + | 1127 |
| Fucose | - | 244 |
| Galactose | + | 1597 |
| Galacturonic Acid | + | 1219 |
| Gentiobiose | + | 1558 |
| Gluconic Acid | + | 1236 |
| Glucose | + | 1569 |
| Inositol | + | 540 |
| Lactose | + | 1336 |
| Lactulose | + | 1184 |
| Maltose | + | 1531 |
| Maltotriose | + | 1544 |
| Mannitol | - | 188 |
| Mannose | + | 1613 |
| Melezitose | + | 1066 |
| Melibiose | + | 1224 |
| Methyl D-Galactoside | + | 661 |
| Methyl D-Galactoside | + | 752 |
| Methyl-D-Glucose | + | 1195 |
| Methyl-D-Glucoside | + | 1277 |
| Methyl-D-Glucoside | + | 1202 |
| Methyl-D-Mannoside | + | 1396 |
| Palatinose | + | 1508 |
| Psicose | + | 1301 |
| Raffinose | + | 1048 |
| Rhamnose | + | 851 |
| Ribose | + | 898 |
| Salicin | + | 1493 |
| Sedoheptulosan | + | 1107 |
| Sorbitol | + | 1226 |
| Stachyose | + | 863 |
| Sucrose | + | 1507 |
| Tagatose | + | 1637 |
| Trehalose | + | 1511 |
| Turanose | + | 1439 |
| Xylitol | + | 1431 |
| Xylose | + | 1595 |
| Acetic Acid | - | 224 |
| Hydroxybutyric Acid | + | 1435 |
| Hydroxybutyric Acid | + | 1042 |
| Hydroxybutyric Acid | - | 243 |
| Hydroxyphenyl-acetic Acid | - | -197 |
| Ketoglutaric Acid | + | 1035 |
| Ketovaleric Acid | + | 434 |
| Lactamide | + | 885 |
| Lactic Acid Methyl Ester | + | 1313 |
| Lactic Acid | + | 1478 |
| Malic Acid | + | 1108 |
| Malic Acid | + | 824 |
| Pyruvic Acid Methyl Ester | + | 1221 |
| Succinic Acid Mono-Methyl Ester | + | 1187 |
| Propionic Acid | + | 370 |
| Pyruvic Acid | + | 1232 |
| Succinamic Acid | + | 1178 |
| Succinic Acid | + | 509 |
| Acetyl-L-Glutamic Acid | + | 1320 |
| Alaninamide | + | 1420 |
| Alanine | - | -10 |
| Alanine | - | 286 |
| Alanyl-Glycine | + | 725 |
| Asparagine | + | 1080 |
| Glutamic Acid | + | 1113 |
| Glycyl-L-Glutamic Acid | + | 1035 |
| Pyroglutamic Acid | + | 1229 |
| Serine | + | 1273 |
| Putrescine | + | 1065 |
| Butanediol | - | 348 |
| Glycerol | + | 1294 |
| Adenosine | + | 1180 |
| Deoxy Adenosine | + | 1011 |
| Inosine | + | 1492 |
| Thymidine | + | 1141 |
| Uridine | + | 1165 |
| Adenosine-5'-Monophosphate | + | 1112 |
| Thymidine-5'-Monophosphate | + | 1029 |
| Uridine 5'-Monophosphate | + | 1085 |
| Fructose-6-Phosphate | + | 1066 |
| Glucose-1-Phosphate | + | 1375 |
| Glucose-6-Phosphate | + | 1368 |
| Glycerol Phosphate | + | 1339 |

Strain *Stenotrophomonas maltophilia UI3E* (DSM 27225): profilo metabolico

**MicroPlate GN2**

| Substrate | Reaction | (OD) |
|---|---|---|
| Water | - | 0 |
| α-Cyclodextrin | - | 3 |
| Dextrin | + | 392 |
| Glycogen | - | 11 |
| Tween 40 | + | 245 |
| Tween 80 | + | 206 |
| N-Acetyl-D-Galactosamine | + | 520 |
| N-Acetyl-D-Glucosamine | + | 496 |
| Adonitol | - | 5 |
| L- Arabinose | - | 26 |
| D-Arabitol | - | -1 |
| D-Cellobiose | + | 493 |
| i-Erythritol | - | 0 |
| D-Fructose | + | 492 |
| L-Fucose | - | 4 |
| D-Galactose | + | 584 |
| Gentiobiose | + | 487 |
| α-D-Glucose | + | 460 |
| m-Inositol | - | 1 |
| α-D-Lactose | + | 405 |
| Lactulose | + | 483 |
| Maltose | + | 501 |
| D-Mannitol | - | 2 |
| D-Mannose | + | 549 |
| D-Melibiose | + | 369 |
| β-Methyl-D-Glucoside | + | 462 |
| D-Psicose | + | 297 |
| D-Raffinose | - | 12 |
| L-Rhamnose | - | 3 |
| D-Sorbitol | - | 3 |
| Sucrose | + | 489 |
| D-Trehalose | + | 458 |
| Turanose | + | 503 |
| Xylitol | - | 3 |
| Pyruvic Acid Methyl Ester | + | 370 |
| Succinic Acid Mono-Methyl Ester | + | 437 |
| Acetic Acid | + | 463 |
| Cis-Aconitic Acid | + | 397 |
| Citric Acid | + | 410 |
| Formic Acid | B | 67 |
| D-Galactonic Acid Lactone | - | 4 |
| D-Galacturonic Acid | - | 8 |
| D-Gluconic Acid | - | 6 |
| D-Glucosaminic Acid | - | 1 |
| D-Glucuronic Acid | - | 7 |
| α-Hydroxybutyric Acid | + | 355 |
| β-Hydroxybutyric Acid | + | 432 |
| γ-Hydroxybutyric Acid | - | 8 |
| p-Hydroxyphenyl-acetic Acid | | 4 |
| Itaconic Acid | - | 1 |
| α-Ketobutyric Acid | + | 356 |
| α-Ketoglutaric Acid | + | 421 |
| α-Ketovaleric Acid | + | 289 |
| D,L-Lactic Acid | + | 395 |
| Malonic Acid | + | 379 |
| Propionic Acid | + | 374 |
| Quinic Acid | - | 2 |
| D-Saccharic Acid | - | 3 |
| Sebacic Acid | - | 5 |
| Succinic Acid | + | 457 |
| Bromosuccinic Acid | + | 487 |
| Succinamic Acid | - | 9 |
| Glucuronamide | - | 3 |
| L-Alaninamide | + | 205 |
| D-Alanine | + | 260 |
| L-Alanine | + | 248 |
| L-Alanyl-Glycine | + | 274 |
| L-Asparagine | B | 86 |
| L-Aspartic Acid | B | 113 |
| L-Glutamic Acid | - | 19 |
| Glycyl-L-Aspartic Acid | + | 338 |
| Glycyl-L-Glutamic Acid | + | 369 |
| L-Histidine | B | 83 |
| Hydroxy-L-Proline | - | 3 |
| L-Leucine | + | 216 |
| L-Ornithine | - | 8 |
| L-Phenylalanine | - | 3 |
| L-Proline | + | 301 |
| L-Pyroglutamic Acid | - | -4 |
| D-Serine | - | 0 |
| L-Serine | + | 382 |
| L-Threonine | + | 290 |
| D,L-Carnitine | - | -1 |
| g-Aminobutyric Acid | - | 1 |
| Urocanic Acid | - | 17 |
| Inosine | + | 250 |
| Uridine | + | 319 |
| Thymidine | - | 0 |
| Phenylethylamine | - | -2 |
| Putrescine | - | -2 |
| 2-Aminoethanol | - | 0 |
| 2,3-Butanediol | - | 1 |
| Glycerol | - | 1 |
| D,L-α-Glycerol Phosphate | - | 2 |
| α-D-Glucose-1-Phosphate | - | 6 |
| D-Glucose-6-Phosphate | - | 10. |

## Claims

1. A method for removing coherent deposits of various nature, both organic and inorganic, also superimposed, from art works and/or wall paintings, **characterized in that** it comprises the following steps:
a) using one or more original non-spore-forming bacterial strains, aerobic, selected among: *Stenotrophomonas maltophilia UI3E* DSM 27225, *Cellulomonas sp. THF11E* DSM 27224, *Pseudomonas koreensis UT30E* DSM 27226, immobilized into an inert support of gelling synthetic clay having formula Na_{0.7}[(Si₈Mg_{5.5}Li_{0.3})O₂₀(OH)₄], known with the commercial name of laponite (Laponite®RD Rockwood Additives, CAS No. 53320-86-8);
b) applying an uniform layer of such laponite gel containing one or more of said bacterial strains on the surface to be treated and allowing to react for at least 24 hours at room temperature;
c) removing the gel from the treated surface;
d) removing the coherent deposits, reduced and softened by the gel, with a water moistened sponge and a brush.

2. Method for removing coherent deposits of various nature, both organic and inorganic, also superimposed, from art works and/or wall paintings, **characterized in that** it comprises the following steps:
Step A: detailed analysis of the chemical composition of the deposits present on the surface to be treated.
Step B: incubation of one or more of the bacterial strains selected depending on the type of metabolic action necessary to perform the better deposits removal, including: *Stenotrophomonas maltophilia UI3E* DSM 27225, *Cellulomonas sp. TBF11E* DSM 27224, *Pseudomonas koreensis UT30E* DSM 27226 in the TSB culture medium in constant agitation at 150 rpm at 28 °C, until reaching the maximum optical density value.
Step C: centrifugation of liquid bacterial cultures.
Step D: resuspension of the cells contained in the pellet in a liquid medium with variable composition depending on the strain and the type of application which it is intended for, according to the following scheme:
D1) for the removal of organic deposits, namely casein, the strain *Stenotrophomonas maltophilia UI3E* DSM 27225 is resuspended in a solution of sodium pyrophosphate 0.5 % w/v;
D2) for the removal of carbonate and gypsum deposits, the strain *Cellulomonas sp. TBF11E* DSM 27224 is resuspended in TSB culture medium diluted 1:10 v/v with deionized water.
D3) for the removal of phosphate deposits, the strain *Pseudomonas koreensis UT30E* DSM 27226 is resuspended in TSB culture medium diluted 1:10 v/v with deionized water.
Step E: addition of laponite to cell suspensions at a concentration ranging between 5 and 15 % w/v.
Step F: incubation of the gel containing the bacterial cells at a temperature of 4 °C for 16-20 hours.
Step G: application, using a spatula, of the obtained gel on the surfaces to be treated for at least 24 hours at temperature between 6 and 37 °C.
Step H: removal of the gel by a spatula.
Step I: removal of the coherent deposits, reduced and softened by the gel, with a water moistened sponge and a brush.

3. Method for removing coherent deposits of various nature from art works and/or wall paints, according to claim 2, **characterized in that** in step E the laponite is added to the cell suspension at a concentration between 7 and 11% w/v.

4. Method for removing coherent deposits of various nature, from art works and/or wall paintings, according to claim 2, **characterized in that** in step E the laponite is added to the cell suspension at a concentration of 9 % w/v.

5. Use of bacterial strains *Stenotrophomonas maltophilia UI3E* DSM 27225, *Cellulomonas sp. TBF11E* DSM 27224, *Pseudomonas koreensis UT30E,* DSM 27226, individually or in combination, immobilized within an inert support of laponite gel for the removal of coherent deposits of different nature from art works and/or wall paintings.

6. Use of the bacterial strain *Stenotrophomonas maltophilia UI3E* DSM 27225 according to claim 5 for the removal of organic deposits, specifically casein, from art works and/or wall paintings, in physiological starvation state.

7. Use of the bacterial strain *Cellulomonas sp. THF11E* DSM 27224 according to claim 5 for the removal of carbonate and gypsum deposits from art works and/or wall paintings.

8. Use of the bacterial strain *Pseudomonas koreensis UT30E* DSM 27226 according to claim 5 for the removal of phosphate-based deposits from art works and/or wall paintings.

9. A biological culture of one or more microorganisms effective in a process of removal of coherent deposits of various nature, organic and inorganic, from art works and/or wall paintings, **characterized in that** said microorganisms are selected from the group consisting of: *Stenotrophomonas maltophilia UI3E* deposited at the Deutsche Sammlung von Mikroorganism Und Zellkulturen GmbH, Braunschweig, Germany, with identification number DSM 27225, *Cellulomonas sp. TBF11E* deposited at the Deutsche Sammlung von Mikroorganism Und Zellkulturen GmbH, Braunschweig, Germany with identification number DSM 27224, *Pseudomonas koreensis UT30E* deposited at the Deutsche Sammlung von Mikroorganism Und Zellkulturen GmbH, Braunschweig, Germany, identified by the number DSM 27226.

10. Biological composition constituted by the association of the bacterial strains according to claim 9, individually or in combination with each other, with an inert matrix in the form of laponite gel.

11. Kit for the bio-cleansing of art works and/or coherent wall deposits comprising:
i) tubes each containing a pure bacterial strain selected from lyophilized: *Stenotrophomonas maltophilia UI3E* DSM 27225, *Cellulomonas sp. TBF11E* DSM 27224, *Pseudomonas koreensis UT30E* DSM 27226;
ii) vials containing aliquots of the culture medium for the optimal growth of the three individual lyophilized strains in amount sufficient for one application;
iii) vials containing aliquots of sterilized deionized water in suitable amount to dissolve the contents of one vial of culture medium;
iv) sachets containing laponite, in sufficient amount for one application;
v) means for applying and removing the microorganisms suspension and laponite on the surface to be treated;
vi) explanatory kit user manual.

## Patentansprüche

1. Verfahren zum Entfernen kohärenter Ablagerungen verschiedener Art, sowohl organisch als auch anorganisch, auch übereinander gelagert, von Kunstwerken und/oder Wandbemalungen, **gekennzeichnet dadurch, dass** es die folgenden Schritte umfasst:
a) Verwenden von einem oder mehreren original nicht Sporen bildenden, aeroben Bakterienstämmen, ausgewählt aus: *Stenotrophomonas maltophilia UI3E* DSM 27225, *Cellulomonas sp. TBF11E* DSM 27224, *Pseudomonas koreensis UT30E* DSM 27226, immobilisiert auf einem inerten Träger aus synthetischem, gellierendem Ton mit der Formel Na_{0,7}[(Si₈Mg_{5,5}Li_{0.3})O₂₀(OH)₄], bekannt unter dem kommerziellen Namen Laponite (Laponite® RD Rockwood Additives, CAS No. 53320-86-8) ;
b) Aufbringen einer gleichmäßigen Schicht des Laponite-Gels, welches einen oder mehrere Bakterienstämme enthält, auf die zu behandelnde Oberfläche und Reagieren lassen für wenigstens 24 Stunden bei Raumtemperatur;
c) Entfernen des Gels von der behandelten Oberfläche;
d) Entfernen der von dem Gel reduzierten und aufgeweichten kohärenten Ablagerungen mit einem mit Wasser befeuchtetem Schwamm und einer Bürste.

2. Verfahren zum Entfernen kohärenter Ablagerungen verschiedener Art, sowohl organisch als auch anorganisch, auch übereinander gelagert, von Kunstwerken und/oder Wandbemalungen, **gekennzeichnet dadurch, dass** es die folgenden Schritte umfasst:
Schritt A: Detaillierte Analyse der chemischen Zusammensetzung der vorhandenen Ablagerungen auf der zu behandelnden Oberfläche.
Schritt B: Inkubieren von einem oder mehreren Bakterienstämmen ausgewählt aufgrund des Typs des notwendigen metabolischen Vorgangs um eine bessere Entfernung der Ablagerungen zu leisten, umfassend: *Stenotrophomonas maltophilia UI3E* DSM 27225, *Cellulomonas sp. TBF11E* DSM 27224, *Pseudomonas koreensis UT30E* DSM 27226 in einem TSB Nährmedium bei konstantem Rühren mit 150 UPM bei 28°C, bis ein maximaler optischer Dichtewert erreicht wird.
Schritt C: Zentrifugieren der flüssigen Bakterienkulturen.
Schritt D: Resuspension der in einem Pellet enthaltenen Zellen in einem flüssigen Medium mit einer variablen Zusammensetzung, abhängig vom Stamm und von der gewünschten Anwendung, nach folgendem Schema:
D1) zum Entfernen von organischen Ablagerungen, hauptsächlich Casein, wird der Stamm *Stenotrophomonas maltophilia UI3E* DSM 27225, in einer 0,5 % w/v Natriumpyrophosphat-Lösung resuspendiert;
D2) zum Entfernen von Carbonat- und Gipsablagerungen wird der Stamm *Cellulomonas sp. TBF11E* DSM 27224 in einem mit entionisiertem Wasser 1:10 v/v verdünntem TSB Nährmedium resuspendiert.
D3) zum Entfernen von Phosphatablagerungen wird der Stamm *Pseudomonas koreensis UT30E* DSM 27226 in einem mit entionisiertem Wasser 1:10 v/v verdünntem TSB Nährmedium resuspendiert.
Schritt E: Zugabe von Laponite zur Zellsuspension in einer Konzentration im Bereich zwischen 5 und 15 % w/v.
Schritt F: Inkubieren des Gels, welches die Bakterienzellen enthält, bei einer Temperatur von 4°C für 16-20 Stunden.
Schritt G: Aufbringen des erhaltenen Gels unter Verwendung eines Spatels auf die zu behandelnde Oberfläche für wenigstens 24 Stunden bei einer Temperatur zwischen 6 und 37 °C.
Schritt H: Entfernen des Gels mit einem Spatel.
Schritt I: Entfernen der von dem Gel reduzierten und aufgeweichten kohärenten Ablagerungen mit einem mit Wasser befeuchtetem Schwamm und einer Bürste.

3. Verfahren zum Entfernen kohärenter Ablagerungen verschiedener Art von Kunstwerken und/oder Wandbemalungen nach Anspruch 2, **gekennzeichnet dadurch, dass** in Schritt E das Laponite zur Zellsuspension in einer Konzentration zwischen 7 und 11% w/v zugegeben wird.

4. Verfahren zum Entfernen kohärenter Ablagerungen verschiedener Art von Kunstwerken und/oder Wandbemalungen nach Anspruch 2, **gekennzeichnet dadurch, dass** in Schritt E das Laponite zur Zellsuspension in einer Konzentration von 9 % w/v zugegeben wird.

5. Verwendung der Bakterienstämme *Stenotrophomonas maltophilia UI3E* DSM 27225, *Cellulomonas sp. TBF11E* DSM 27224, *Pseudomonas koreensis UT30E,* DSM 27226, individuell oder in Kombination, immobilisiert auf einem inerten Träger aus Laponite-Gel zum Entfernen kohärenter Ablagerungen verschiedener Art von Kunstwerken und/oder Wandbemalungen.

6. Verwendung des Bakterienstamms *Stenotrophomonas maltophilia UI3E* DSM 27225 in einem physiologischen Hungerzustand nach Anspruch 5, zum Entfernen von organischen Ablagerungen, speziell Casein, von Kunstwerken und/oder Wandbemalungen.

7. Verwendung des Bakterienstamms *Cellulomonas sp. TBF11E* DSM 27224 nach Anspruch 5, zum Entfernen von Carbonat- und Gipsablagerungen von Kunstwerken und/oder Wandbemalungen.

8. Verwendung des Bakterienstamms *Pseudomonas koreensis* UT30E DSM 27226 nach Anspruch 5, zum Entfernen von Phosphatbasierten Ablagerungen von Kunstwerken und/oder Wandbemalungen.

9. Biologische Kultur aus einem oder mehreren Mikroorganismen, effektiv im Prozess des Entfernens von kohärenten Ablagerungen verschiedener Art, sowohl organisch als auch anorganisch, auch übereinander gelagert, von Kunstwerken und/oder Wandbemalungen, **gekennzeichnet dadurch, dass** die Mikroorganismen ausgewählt werden aus der Gruppe bestehend aus: *Stenotrophomonas maltophilia UI3E,* gelagert in der Deutschen Sammlung von Mikroorganismen und Zellkulturen GmbH, Braunschweig, Deutschland, mit der Identifikationsnummer DSM 27225, *Cellulomonas sp. TBF11E,* gelagert in der Deutschen Sammlung von Mikroorganismen und Zellkulturen GmbH, Braunschweig, Deutschland, mit der Identifikationsnummer DSM 27224, *Pseudomonas koreensis UT30E,* gelagert in der Deutschen Sammlung von Mikroorganismen und Zellkulturen GmbH, Braunschweig, Deutschland, identifiziert durch die Nummer DSM 27226.

10. Biologische Zusammensetzung zusammengesetzt aus der Verbindung der Bakterienstämme nach Anspruch 9, individuell oder in Kombination miteinander, mit einer inerten Matrix in Form eines Laponite-Gels.

11. Kit zur Bio-Reinigung von Kunstwerken und/oder kohärenten Wandablagerungen umfassend:
i) Röhren die jeweils einen reinen Bakterienstamm enthalten, ausgewählt aus lyophilisierten: *Stenotrophomonas maltophilia UI3E* DSM 27225, *Cellulomonas sp. TBF11E* DSM 27224, *Pseudomonas koreensis UT30E* DSM 27226;
ii) Fläschchen, welche Aliquote der Nährmedien für ein optimales Wachstum der drei individuellen lyophilisierten Stämme in einer für eine Anwendung ausreichenden Menge enthalten;
iii) Fläschchen, welche Aliquote von sterilisiertem, entionisiertem Wasser in einer passenden Menge zum Verdünnen des Inhalts eines Fläschchens an Nährmedium enthalten;
iv) Portionsbeutel, welche Laponite in einer für eine Anwendung ausreichenden Menge enthalten;
v) Mittel zum Anwenden und Entfernen der Suspension an Mikroorganismen und Laponite von der zu behandelnden Oberfläche;
vi) erläuternde Kit-Bedienungsanleitung.

## Revendications

1. Procédé pour l'élimination de dépôts cohérents de différente nature, à la fois organique et inorganique, également superposés, d'oeuvres d'art et/ou de peintures murales, **caractérisé en ce qu'**il comprend les étapes suivantes :
a) l'utilisation d'une ou plusieurs souches bactériennes non sporulantes originales, aérobies, sélectionnées parmi : Stenotrophomonas maltophilia UI3E DSM 27225, Cellulomonas sp. TBF11E DSM 27224, Pseudomonas koreensis UT30E DSM 27226, immobilisées dans un support inerte d'argile synthétique gélifiant ayant la formule Na₀,₇[(Si₈Mg_{5,5}Li_{0,3})O₂₀(OH)₄], connu sous le nom commercial de laponite (Laponite®RD Rockwood Additives, CAS n° 53320-86-8) ;
b) l'application d'une couche uniforme de ce gel de laponite contenant une ou plusieurs desdites souches bactériennes sur la surface à traiter et le fait de laisser réagir pendant au moins 24 heures à température ambiante ;
c) l'élimination du gel de la surface traitée ;
d) l'élimination des dépôts cohérents, réduits et ramollis par le gel, avec une éponge humidifiée à l'eau et une brosse.

2. Procédé pour l'élimination de dépôts cohérents de différente nature, à la fois organique et inorganique, également superposés, d'oeuvres d'art et/ou de peintures murales, **caractérisé en ce qu'**il comprend les étapes suivantes :
Etape A : l'analyse détaillée de la composition chimique des dépôts présents sur la surface à traiter.
Etape B : l'incubation d'une ou plusieurs des souches bactériennes sélectionnées en fonction du type d'action métabolique nécessaire pour effectuer la meilleure élimination de dépôts, comprenant : Stenotrophomonas maltophilia UI3E DSM 27225, Cellulomonas sp. TBF11E DSM 27224, Pseudomonas koreensis UT30E DSM 27226 dans le milieu de culture TSB en agitation constante à 150 tours/minute à 28°C, jusqu'à atteindre la valeur de densité optique maximale.
Etape C : la centrifugation de cultures bactériennes liquides.
Etape D : la remise en suspension des cellules contenues dans les granulés dans un milieu liquide avec une composition variable en fonction de la souche et du type d'application à laquelle elle est destinée, selon le schéma suivant :
D1) pour l'élimination de dépôts organiques, notamment la caséine, la souche Stenotrophomonas maltophilia UI3E DSM 27225 est remise en suspension dans une solution de pyrophosphate de sodium à 0,5% en poids/volume ;
D2) pour l'élimination de dépôts de carbonates et de gypse, la souche Cellulomonas sp. TBF11E DSM 27224 est remise en suspension dans un milieu de culture TSB dilué à 1:10 en volume avec de l'eau désionisée.
D3) pour l'élimination de dépôts de phosphates, la souche Pseudomonas koreensis UT30E DSM 27226 est remise en suspension dans un milieu de culture TSB dilué à 1:10 en volume avec de l'eau désionisée.
Etape E : l'ajout de laponite à des suspensions cellulaires à une concentration dans la plage entre 5 et 15% en poids/volume.
Etape F : l'incubation du gel contenant les cellules bactériennes à une température de 4°C pendant 16 à 20 heures.
Etape G : l'application, en utilisant une spatule, du gel obtenu sur les surfaces à traiter pour au moins 24 heures à une température entre 6 et 37°C.
Etape H : l'élimination du gel avec une spatule.
Etape I : l'élimination des dépôts cohérents, réduits et ramollis par le gel, avec une éponge humidifiée à l'eau et une brosse.

3. Procédé pour l'élimination de dépôts cohérents de différente nature d'oeuvres d'art et/ou de peintures murales selon la revendication 2, **caractérisé en ce que**, dans l'étape E, la laponite est ajoutée à la suspension cellulaire à une concentration entre 7 et 11% en poids/volume.

4. Procédé pour l'élimination de dépôts cohérents de différente nature d'oeuvres d'art et/ou de peintures murales selon la revendication 2, **caractérisé en ce que**, dans l'étape E, la laponite est ajoutée à la suspension cellulaire à une concentration de 9% en poids/volume.

5. Utilisation de souches bactériennes Stenotrophomonas maltophilia UI3E DSM 27225, Cellulomonas sp. TBF11E DSM 27224, Pseudomonas koreensis UT30E DSM 27226, individuellement ou en combinaison, immobilisées dans un support inerte de gel de laponite pour l'élimination de dépôts cohérents de différente nature d'oeuvres d'art et/ou de peintures murales.

6. Utilisation de la souche bactérienne Stenotrophomonas maltophilia UI3E DSM 27225 selon la revendication 5 pour l'élimination de dépôts organiques, spécifiquement la caséine, d'oeuvres d'art et/ou de peintures murales, dans un état de carence physiologique.

7. Utilisation de la souche bactérienne Cellulomonas sp. TBF11E DSM 27224 selon la revendication 5 pour l'élimination de dépôts de carbonates et de gypse d'oeuvres d'art et/ou de peintures murales.

8. Utilisation de la souche bactérienne Pseudomonas koreensis UT30E DSM 27226 selon la revendication 5 pour l'élimination de dépôts à base de phosphates d'oeuvres d'art et/ou de peintures murales.

9. Culture biologique d'un ou plusieurs microorganismes efficaces dans un processus d'élimination de dépôts cohérents de différente nature, organique et inorganique, d'oeuvres d'art et/ou de peintures murales, **caractérisé en ce que** lesdits microorganismes sont sélectionnés à partir du groupe consistant en : Stenotrophomonas maltophilia UI3E déposée auprès de la Deutsche Sammlung von Mikroorganism Und Zellkulturen GmbH, Braunschweig, Allemagne, avec le numéro d'identification DSM 27225, Cellulomonas sp. TBF11E déposée auprès de la Deutsche Sammlung von Mikroorganism Und Zellkulturen GmbH, Braunschweig, Allemagne, avec le numéro d'identification DSM 27224, Pseudomonas koreensis UT30E déposée auprès de la Deutsche Sammlung von Mikroorganism Und Zellkulturen GmbH, Braunschweig, Allemagne, identifiée par le numéro DSM 27226.

10. Composition biologique constituée par l'association des souches bactériennes selon la revendication 9, individuellement ou en combinaison entre elles, avec une matrice inerte sous la forme de gel de laponite.

11. Kit pour le bio-nettoyage de dépôts cohérents muraux et/ou d'oeuvres d'art comprenant :
i) des tubes contenant chacun une souche bactérienne pure sélectionnée à partir de : Stenotrophomonas maltophilia UI3E DSM 27225, Cellulomonas sp. TBF11E DSM 27224, Pseudomonas koreensis UT30E DSM 27226, lyophilisée ;
ii) des fioles contenant des parties aliquotes du milieu de culture pour la croissance optimale des trois souches lyophilisées individuelles en quantité suffisante pour une application ;
iii) des fioles contenant des parties aliquotes d'eau désionisée stérilisée en quantité suffisante pour dissoudre le contenu d'une fiole de milieu de culture ;
iv) des sachets contenant de la laponite, en quantité suffisante pour une application ;
v) des moyens pour appliquer et éliminer la suspension de microorganismes et la laponite sur la surface à traiter ;
vi) un manuel d'utilisateur de kit explicatif.
